(19) Europäisches Patentamt
European Patent Office
Office européen des brevets

(11) Veröffentlichungsnummer: **0 118 095**
**B1**

(12) # EUROPÄISCHE PATENTSCHRIFT

(45) Veröffentlichungstag der Patentschrift: **30.03.88**

(51) Int. Cl.⁴: **C 07 C 67/11**, C 07 C 69/94, C 07 C 51/42, C 07 C 65/11

(21) Anmeldenummer: **84102057.1**

(22) Anmeldetag: **28.02.84**

(54) **Verfahren zur Herstellung von reinen 1-Hydroxy-2-naphthoesäurealkylestern.**

(30) Priorität: **04.03.83 DE 3307637**

(43) Veröffentlichungstag der Anmeldung:
**12.09.84 Patentblatt 84/37**

(45) Bekanntmachung des Hinweises auf die Patenterteilung:
**30.03.88 Patentblatt 88/13**

(84) Benannte Vertragsstaaten:
**BE CH DE FR GB LI NL**

(56) Entgegenhaltungen:
**EP - A - 0 097 926**
**DE - A - 1 643 541**
**DE - A - 2 407 187**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher-Strasse 40, D-6000 Frankfurt am Main 50 (DE)**
Erfinder: **Volk, Heinrich, Dr., Am weissen Stein 1, D-6368 Bad Vilbel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 118 095 B1

ACTORUM AG

**Beschreibung**

1-Hydroxy-2-naphthoesäure-alkylester sind wertvolle Zwischenprodukte zur Herstellung von Farbstoffen und insbesondere von farbgebenden Schichten in fotografischen Materialien, wobei sie bei der Herstellung sogenannter «Instantfilme» zur Erzeugung von Sofortbildern und -filmen technisches Interesse erwecken konnten.

Vornehmlich für das letztgenannte Einsatzgebiet werden farblose, kristalline Verbindungen der Formel

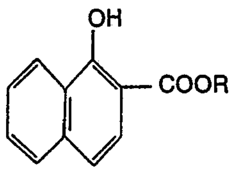

in welcher R eine Methyl-, Äthyl-, n- oder iso-Propyl- oder n- oder iso-Butylgruppe bedeutet, benötigt, die frei von jeglichen Verunreinigungen, insbesondere von synthesebedingten Verunreinigungen aus den Vorstufen, sind, beispielsweise frei von 1-Hydroxy-naphthoesäure, 1-Alkoxynaphthoesäure und 1-Alkoxynaphthoesäure-alkylestern, sowie vor allem von höhermolekularen, harzartigen Verbindungen, wie sie bei der technischen Herstellung von 1-Hydroxynaphthoesäure durch Carboxylierung von 1-Naphtholaten mit Kohlendioxid nach Kolbe-Schmitt zwangsweise entstehen, und weder im Isolierprozess der Hydroxynaphthoesäure, noch aus ihren Folgestufen bis heute quantitativ entfernt werden können.

Während die erstgenannten Verunreinigungen durch spezielle Synthesebedingungen in ihrer Bildung auf ein Minimum reduziert werden können und durch Umkristallisieren der Endprodukte der genannten Formel I vollständig entfernt werden können, trifft dies für die letztgenannten harzartigen höhermolekularen Verunreinigungen nicht zu. Selbst mehrmaliges Umkristallisieren der Endprodukte der genannten Formel (I) mit oder ohne Zusatz von Adsorbentien, Klärhilfsmitteln und/oder Reduktions- oder Oxidationsmitteln, liefert in keinem Falle farblose, analysenreine 1-Hydroxynaphthoesäure-2-alkylester, wie sie für die Herstellung von Sofortbildfilmen unabdingbar sind. Es mussten daher bisher aufwendige chromatografische Verfahren zur Erzielung der erforderlichen Qualität (Reinheit) durchgeführt werden.

Es wurde nun überraschenderweise gefunden, dass man reine 1-Hydroxy-2-naphthoesäurealkylester der allgemeinen Formel (I)

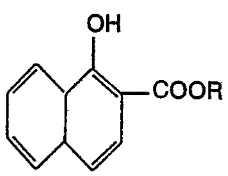 (I),

in welcher R eine geradkettige oder verzweigte Alkylgruppe von 1 bis 5 Kohlenstoffatomen bedeutet, in hoher Ausbeute auf technisch einfache Weise herstellen kann, indem man eine wässrige Lösung der Mono- und/oder Di-alkalimetallsalze der 1-Hydroxy-2-naphthoesäure, erhalten durch Carboxylierung eines Alkalimetall-1-nahptholats [nach Kolbe-Schmitt; vergleiche hierzu BIOS Final Report. Nr. 986, Seiten 234–249 und 219–225; HOUBEN WEYL 8, 372 ff (1952); H. Kolbe, LIEBIGS ANNALEN 113, 125 (1860); R. Schmitt, Berichte der Deutsch. chem. Gesellschaft 20, 2702 (1887)] und nachfolgende Behandlung mit Wasser oder durch Auflösen einer auf diese Weise hergestellten, jedoch zwischenisolierten 1-Hydroxy-2-naphthoesäure in wässriger Alkalilauge, mittels Mineralsäure auf einen pH-Wert von 5,5 bis 6,5, vorzugsweise 6,0 einstellt, dann eine oberflächenaktive, kationische Verbindung.

(1) der allgemeinen Formel (II)

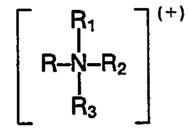 $X^{(-)}$ (II),

in welcher R, $R_1$, $R_2$ und $R_3$ gleiche oder verschiedene, geradkettige oder verzweigte, gesättigte oder 1, 2 oder 3 Kohlenstoffdoppelbindungen enthaltende aliphatische Reste von 1 bis 30 Kohlenstoffatomen, die 1 bis 3 Heteroatome oder Gruppierungen aus der Reihe

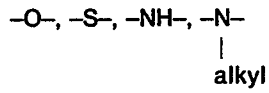

oder quaternierte Stickstoffatome, und/oder Carbonsäureamidgruppen enthalten können, und die ferner an den Kettenenden ionogene Substituenten, wie beispielsweise Hydroxy- oder Alkoxy- oder Polyglykoläthergruppen von vorzugsweise 1 bis 30 Kohlenstoffatomen enthalten können, oder hydroaromatische carbocyclische oder cycloaliphatische Reste von jeweils 4 bis 8 Ringkohlenstoffatomen, wobei diese cyclischen Reste aliphatische Seitenketten von 1 bis 12 Kohlenstoffatomen oder Halogenatome, wie beispielsweise Chlor- oder Bromatome, tragen können, oder Aralkylreste, wobei der Alkylrest 1 bis 30 Kohlenstoffatome, vorzugsweise 1 bis 12 Kohlenstoffatome enthält, und der Arylrest einen Phenyl- oder Naphthylrest darstellt, der am aromatischen Kern durch Hydroxygruppen, niedere Alkylgruppen, niedere Alkoxygruppen und/oder Halogenatome, wie Chloratome, substituiert sein kann, oder Arylreste, vorzugsweise Phenyl- oder Naphthylreste, die am aromatischen Kern durch Hydroxy-, niedere Alkoxy-, niedere Alkyl-, Carbonamid- oder Sulfonamidgruppen oder Halogenatome, wie beispielsweise Chloratome, substituiert sein können, bedeuten, wobei die Summe der Kohlenstoffatome von R, $R_1$, $R_2$ und $R_3$ mindestens 8 beträgt, und wobei zwei oder drei der am quartären Stickstoffatom befindlichen aliphatischen Reste R, $R_1$, $R_2$ und $R_3$ gemeinsam mit dem Stickstoffatom einen heterocyclischen, ggfs. Doppelbindungen enthaltenden Ring, an welchen ein Benzolkern ankondensiert sein kann, bilden können, wie beispielsweise eine Pyridin-, Morpholin-, Imidazolin-, Benzimidazolin-, Imidazol-, Benzimidazol- oder Oxazolring, und in welcher $X^{(-)}$ das Äquivalent einer anorganischen oder organischen Säure darstellt, oder

(2) der allgemeinen Formel (III)

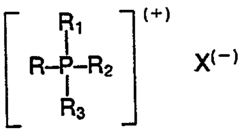

(III),

in welcher R, $R_1$, $R_2$, $R_3$ und $X^{(-)}$ die bei der Definition der Formel (II) genannten Bedeutungen haben, wobei die Summe der Kohlenstoffatome von R, $R_1$, $R_2$ und $R_3$ mindestens 8 ist und zwei oder drei der aliphatischen Reste R, $R_1$, $R_2$ und $R_3$ mit dem Phosphoratom einen heterocyclischen, ggfs. Doppelbindungen enthaltenden Ring bilden können, jeweils in einer Menge von 0,5 bis 5 Gew.-%, vorzugsweise 0,5 bis 3 Gew.-%, bezogen auf die in der Lösung vorliegende Menge an 1-Hydroxy-2-naphthoesäure, zugibt und die sich dabei in fester oder flüssiger Form ausscheidenden, aus den vorhandenen Harzen in quantitativer Reaktion gebildeten Niederschläge bei Temperaturen von 50 °C bis 100 °C, vorzugsweise 70 °C–80 °C, ggfs. nach Zugabe von Adsorbentien, wie Aktivkohle und/oder Filterhilfsmitteln, wie dispersen Silikaten oder Aluminiumoxiden, separiert oder abfiltriert und das in wässriger Lösung vorliegende reine Mono-Alkalimetallsalz der 1-Hydroxy-2-naphthoesäure mit Dialkylsulfat im pH-Bereich 5,5 bis 6,5, vorzugsweise 6,0, bei Temperaturen zwischen 30° und 80 °C, vorzugsweise 40° bis 60 °C, verestert.

Bevorzugte Verbindungen der genannten Formel (II) sind solche, bei welchen R ein Alkylrest von 8 bis 20 Kohlenstoffatomen ist, $R_1$ für einen Alkylrest von 1 bis 20 Kohlenstoffatomen, für einen Hydroxyalkylrest mit 2 bis 6 Kohlenstoffatomen oder für einen Phenylalkylrest mit 4 bis 12 Kohlenstoffatomen im Alkylrest steht, $R_2$ ein Alkylrest von 1 bis 8 Kohlenstoffatomen oder ein Hydroxyalkylrest von 2 bis 6 Kohlenstoffatomen ist, und $R_3$ für einen Alkylrest von 1 bis 8 Kohlenstoffatomen oder den Benzylrest steht.

Erfindungsgemäss verwendbare quartäre Ammoniumverbindungen der genannten allgemeinen Formel (II) sind beispielsweise folgende:

Dodecyl-dimethyl-benzyl-ammoniumchlorid,
Oleyl-trimethyl-ammoniumchlorid,
Distearyl-dimethyl-ammoniumchlorid,
Lauryl-dimethylhydroxyäthyl-ammoniumchlorid,
Dodecyl-di-(hydroxyäthyl)-methylammoniumchlorid,
Dodecyl-dimethyl-vinyl-ammoniumchlorid,
Dodecyl-methyl-morpholiniumchlorid,
Lauryl-pyridinumchlorid,
Hexadecyl-N,N′-dimethyl-benzimidazoliniumsulfat,
Dodecyl-di-(triäthylenglykoläther)-benzylammoniumchlorid,
Phenylnonyl-dimethyl-benzyl-ammoniumchlorid,
Oleyl-di-(hydroxyläthyl)-äthylenglykolätherammoniumchlorid,
Oleyl-dimethyl-hydroxyäthyl-ammoniumchlorid,
Cocos-di-(triäthylenglykoläther)-benzylammoniumchlorid,
Cocos-dimethyl-benzyl-ammoniumchlorid,
Distearyl-dimethyl-ammoniumchlorid,
Trioctyl-methyl-ammoniumchlorid,
Cocos-dimethyl-hydroxypropyl-ammoniumchlorid,
p-(iso-Butyl)-phenoxyäthoxyäthyl-dimethylbenzyl-ammoniumchlorid,
Oleyl-methyl-imidazoliniumchlorid,
Hexadecyl-N,N′-dimethyl-benzimidazoliniumsulfat,
Oleyl-methyl-imidazoliumchlorid,
Dehydro-abietyl-dimethyl-benzyl-ammoniumchlorid.

(Unter dem Rest «Cocos» wird ein aliphatischer Rest aus einem Gemisch von Alkyl-, Alkenyl- und Alk-dienyl-Resten von jeweils 8 bis 18 Kohlenstoffatomen verstanden).

Erfindungsgemäss einsetzbare Phosphoniumverbindungen der genannten allgemeinen Formel (III) sind beispielsweise folgende:

Trioctyl-methylphosphoniumchlorid,
Phenyl-nonyl-dimethylbenzylphosphoniumchlorid,
Dodecyl-dimethyl-benzylphosphoniumchlorid,
Lauryl-dimethyl-hydroxyethyl-phosphoniumchlorid,
Tridecyl-methylphosphoniumchlorid,
Distearyl-diethylphosphoniumchlorid oder
Oleyl-trimethyl-phosphoniumchlorid.

Die im Zuge des erfindungsgemässen Verfahrens erhaltenen wässrigen Lösungen reiner Mono-Alkalimetallsalze der 1-Hydroxy-2-naphthoesäure können zur Abscheidung verunreinigungsfreier 1-Hydroxy-2-naphthoesäure durch Zugabe von Mineralsäuren verwendet werden, wobei die abgeschiedene, neutral gewaschene und getrocknete 1-Hydroxy-2-naphthoesäure wieder in Natronlauge gelöst und verfahrensgemäss mit Dialkylsulfat verestert werden kann. Für die erfindungsgemässe Weiterverarbeitung durch Veresterung mittels Dialkylsulfaten wird jedoch die wässrige Lösung bevorzugt direkt eingesetzt, wodurch der zusätzliche Isolierungsschritt entfällt und dabei eine mögliche Qualitätsverschlechterung (beispielsweise durch Waschen mit technischem Waser, ggf. Lagerung und/oder Trocknung) vermieden wird. Die Veresterung wird zweckgemäss in der Weise durchgeführt, dass man die erfindungsgemäss von Harzen befreite wässrige Mono-Alkalimetallsalzlösung der 1-Hydroxy-2-naphthoesäure bei 30°–80 °C, vorzugsweise 40° bis 60 °C und einem pH-Bereich zwischen 5,5 und 6,5, vorzugsweise 6,0, durch gleichzeitigen Zulauf von Dialkylsulfat und einer Alkalihydroxidlösung unter Konstanthaltung des pH-Wertes umsetzt. Der Überschuss an Alkylierungsmittel wird anschliessend durch Zugabe von Ammoniak zerstört und das Endprodukt, gegebenenfalls nach Zwischenklärung mit Aktivkohle und/oder durch Aufhellen mit einem üblichen Bleichmittel, wie Natriumdithionit, mit Mineralsäure bei einem pH-Wert von etwa 7–8,5 ausgefällt, filtriert und ggf. getrocknet.

Als Dialkylsulfate kommen vorzugsweise technisch leicht zugängliche Verbindungen in Frage, wie beispielsweise Dimethylsulfat und Diäthylsulfat.

Gegebenenfalls kann der erfindungsgemäss erhaltene wasserfeuchte oder getrocknete 1-Hydroxy-2-naphthoesäure-alkylester zur Aspektverbesserung (Kristallinität) und/oder weiteren Qualitätssteigerung (Entfernung ggf. enthaltener freier 1-Hydroxy-2-naphthoesäure) aus Alkanolen, vorzugsweise dem jeweils der Alkylestergruppe des Einsatzproduktes entsprechenden Alkanols, umkristallisiert werden.

Man erhält hierbei auf technisch einfache Weise analysenreine, für die Verwendung in fotografischen Schichten hervorragend geeignete 1-Hydroxy-2-naphthoesäure-alkylester in ausgezeichneter Ausbeute, wobei ggf. direkt die durch Carboxylierung von Alkalimetall-1-naphtholat und anschliessende Behandlung mit Wasser erhältliche wässrige Lösung der Alkalimetallsalze der 1-Hydroxy-2-naphthoesäure einsetzbar ist und damit in einer Eintopfreaktion, ausgehend von 1-Naphthol, unter Vermeidung von Regenerationen, Filtrationen, Trocknungen und dazu nötigen aufwendigen Apparaturen die Zielprodukte technisch optimal hergestellt werden können.

Die Veresterung von 1-Hydroxy-2-naphthoesäure mit Dialkylsulfat in alkalischer Lösung ist zwar im Prinzip bekannt (H. Kaufmann, M. Egner, B. 46, 3782 (1913), über Ausbeute und Qualität wurden jedoch in der Literatur keine Angaben gemacht. Erst durch Einhaltung enger, definierter pH- und Temperaturbedingungen wird eine selektive Veresterung der Carboxylgruppe ohne jede Veretherung der konkurrierenden Hydroxygruppe ermöglicht [Luxemburgisches Patent Nr. 71 834].

Allerdings enthalten so hergestellte 1-Hydroxy-2-naphthoesäure-alkylester (neben den im Ausgangsprodukt enthaltenen, nur durch das erfindungsgemässe Verfahren entfernbaren Harzen) immer noch nicht umgesetzte 1-Hydroxy-2-naphthoesäure, von der sie jedoch durch einmaliges Umkristallisieren aus Alkanolen, zweckmässigerweise aus dem der Estergruppe entsprechenden Alkanol, um die Gefahr der Umesterung auszuschliessen, befreit werden können.

Die erfindungsgemäss eingesetzten kationischen, oberflächenaktiven Verbindungen der allgemeinen Formeln II und III ergeben überraschenderweise mit den von der Herstellung her in der 1-Hydroxy-2-naphthoesäure enthaltenen Harzen quantitative, wasserunlösliche Fällungen, die sich, abhängig von den gewählten Fälltemperaturen und abhängig von der Konstitution der oberflächenaktiven Verbindung, in fester oder flüssiger Form bilden, wobei über die Natur der Fällungen der Harze nichts bekannt ist.

Diese quantitativen Fällungen sind insofern als überraschend anzusehen, als gemäss dem Verfahren der deutschen Auslegeschrift 1 643 541 kationische, oberflächenaktive Verbindungen, die unter die vorstehend genannte allgemeine Formel (II) fallen, dazu verwendet werden, um die Phenolat-Carboxylierungen nach dem Vermischen mit Wasser und anschliessendem Ansäuern zur Fällung der gewünschten Hydroxybenzoesäure die in diesen Carboxylierungsproduktgemischen enthaltenen Verunreinigungen in Lösung zu halten und zu vermeiden, dass diese Verunreinigungen zusammen mit der gewünschten Hydroxybenzoesäure ausfallen.

Die nachstehenden Beispiele sollen die vorliegende Erfindung erläutern.

Beispiel 1

Zu 882 Teilen einer dunkelbraunen wässrigen Lösung des Dinatriumsalzes der 1-Hydroxy-2-naphthoesäure, hergestellt durch Carboxylieren von Natrium-1-naphtholat und nachfolgendes Lösen der Carboxylierungsschmelze in Wasser, die 188 Teile freie 1-Hydroxy-2-naphthoesäure enthält, lässt man etwa 100 Teile konzentrierte Schwefelsäure laufen, so dass ein pH-Wert von 5,5 erhalten wird.

Man heizt unter Rühren auf ca. 60 °C, gibt 1,8 Teile Cocos-benzyl-dimethylammoniumchlorid zu und rührt 5 Minuten bei 60 °C nach.

Der ausgefallene schwarzbraune Niederschlag wird abfiltriert und das klare, wasserhelle Filtrat bei 55–60 °C in 3 Stunden mit 277 Teilen Dimethylsulfat versetzt. Durch gleichzeitigen Zulauf von 200 Teilen 33%iger Natronlauge wird der pH-Wert konstant bei 5,5 gehalten. Man rührt 30 Minuten nach, setzt nacheinander 100 Teile Wasser und 22 Teile 25%iges wässriges Ammoniak zu, rührt eine weitere Stunde bei 55–60 °C und kühlt dann auf 20 °C ab.

Der ausgefallene 1-Hydroxy-2-naphthoesäure-methylester wird abgesaugt, mit Wasser neutral gewaschen und getrocknet. Man erhält 195 Teile vom Schmelzpunkt 75,5–76,5 °C, entsprechend einer Ausbeute von 96,5% der Theorie, bezogen auf 1-Hydroxy-2-naphthoesäure (Reingehalt ermittelt durch potentiometrische Titration: 99,8%).

Das so hergestellte farblose kristalline Produkt enthält ausser geringen Mengen 1-Hydroxy-2-naphthoesäure (0,1–0,2%) keinerlei Verunreinigungen. Durch einfaches Umkristallisieren aus der 5–6-fachen Menge Methanol können auch diese quantitativ entfernt werden (Schmelzpunkt dann 76,5–77 °C).

Ersetzt man das Cocos-benzyldimethylammoniumchlorid durch 2,0 Teile Phenylnonyl-dimethylbenzylphosphoniumchlorid und arbeitet sonst wie vorstehend angegeben, so erhält man den 1-Hydroxy-2-naphthoesäuremethylester in vergleichbarer Ausbeute und Qualität.

Vergleichsbeispiel

Arbeitet man, wie in Beispiel 1 beschrieben, lässt aber den Zusatz von Cocos-benzyl-dimethylammoniumchlorid weg, so erhält man in vergleichbarer Ausbeute einen dunkelbraun gefärbten 1-Hydroxy-2-naphthoesäure-methylester vom Schmelzpunkt 69–72 °C (Reingehalt ermittelt

durch potentiometrische Titration: 96,5–97%), der selbst durch 5-maliges, verlustreiches Umkristallisieren aus Methanol unter Zusatz von Aktivkohle nicht farblos und frei von Verunreinigungen erhalten werden kann.

Beispiel 2

188 Teile technische 1-Hydroxy-2-naphthoesäure werden in einer Mischung aus 120 Teilen 33%iger Natronlauge und 520 Teilen Wasser bei 90 °C gelöst. Man kühlt dann auf 75 °C und stellt durch Zugabe von 1,9 Teilen konzentrierter Schwefelsäure auf pH 6.

Zu der dunkelbraunen Lösung werden 4,0 Teile Trioctyl-methylammoniumchlorid zugegeben. Nach 10 Minuten setzt man ausserdem 5 Teile Aktivkohle und 5 Teile eines mineralischen Filterhilfsmittels zu, rührt nochmals 5 Minuten und filtriert über eine vorgewärmte Nutsche. Das farblose klare Filtrat wird auf 40 °C gekühlt. Man lässt in einer Stunde gleichmässig 252 Teile Dimethylsulfat zutropfen, wobei durch gleichzeitigen Zulauf von 120 Teilen 33%iger Natronlauge der pH-Wert konstant bei 6,0 gehalten wird.

Dabei fällt der 1-Hydroxy-2-naphthoesäuremethylester in Form farbloser Kristalle aus. Man rührt 3 Stunden nach, stellt durch Zugabe von 6 Teilen 33%iger Natronlauge auf pH 10,0, rührt eine weitere Stunde nach und kühlt dann auf 20 °C ab. Anschliessend wird abgesaugt, mit Wasser neutral gewaschen und getrocknet. Man erhält 191 Teile 1-Hydroxy-2-naphthoesäure-methylester vom Schmelzpunkt 76–77 °C, der keine nachweisbaren Verunreinigungen enthält. (Reingehalt ermittelt durch potentiometrische Titration: 99,9–100%; Ausbeute: 94,6% d.Th., bezogen auf 1-Hydroxy-2-naphthoesäure). Verwendet man an Stelle der Natronlauge entsprechende Mengen Kalilauge und arbeitet sonst in der angegebenen Weise, so erhält man ein identisches Ergebnis.

Beispiel 3

2000 Volumenteile einer dunkelbraunen wässrigen Lösung aus einer Carboxylierungsschmelze aus der Umsetzung des K-1-naphtholats mit Kohlendioxid, die im Liter Lösung 94 Teile 1-Hydroxy-2-naphthoesäure enthält, werden bei 80 °C mit 30%iger wässriger Salzsäure auf einen pH-Wert von 6,0 eingestellt.

Man gibt 3 Teile Stearyl-dimethyl-benzylammoniumchlorid oder Trioctyl-methylphosphoniumchlorid in Form einer 50%igen wässrigen Lösung zu, rührt 5 Minuten bei 80 °C, gibt dann 6 Teile Aktivkohle zu und klärt die erhaltene Suspension über ein beheiztes Filter. Das klare, farblose wässrige Filtrat wird mit 78%iger Schwefelsäure auf pH 2 eingestellt.

Die dabei ausgefallene 1-Hydroxy-2-naphthoesäure wird abfiltriert, mit Wasser neutral gewaschen und getrocknet. Man erhält 184 Teile farblose 1-Hydroxy-2-naphthoesäure (98% d. Theorie).

94 Teile der so hergestellten 1-Hydroxy-2-naphthoesäure werden in einer Mischung aus 300 Teilen Wasser und 60 Teilen 33%iger Natronlauge bei 35 °C gelöst. Es hat sich ein pH-Wert 6,0–6,5 eingestellt. Durch Zulauf von 150 Teilen Diethylsulfat unter gleichzeitigem Zutropfen von ca. 60 Teilen 33%iger Natronlauge wird innerhalb 3 Stunden bei pH 6,5 und 35 °C Innentemperatur verestert.

Während des Zutropfens scheidet sich der 1-Hydroxy-2-naphthoesäure-ethylester in Form farbloser, grober Kristalle aus.

Man rührt 1 Stunde bei 35 °C nach, zerstört das überschüssige Diethylsulfat durch Zugabe von 3 Teilen 33%iger Natronlauge, kühlt auf 10 °C ab und isoliert das Kristallisat durch Filtration.

Man erhält 210 Teile 1-Hydroxy-2-naphthoesäure-ethylester vom Schmelzpunkt 48,5–49 °C, der keine nachweisbaren Verunreinigungen enthält. (Reingehalt durch potentiometrische Titration: 99,8–99,9%; Ausbeute: 97,2% d.Th., bezogen auf eingesetzte reine 1-Hydroxy-2-naphthoesäure).

**Patentansprüche**

1. Verfahren zur Herstellung reiner 1-Hydroxy-2-naphthoesäurealkylester der allgemeinen Formel (I)

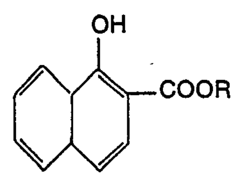

(I),

in welcher R eine geradkettige oder verzweigte Alkylgruppe von 1 bis 5 Kohlenstoffatomen bedeutet, dadurch gekennzeichnet, dass man eine wässrige Lösung der Mono- und/oder Dialkalimetallsalze der 1-Hydroxy-2-naphthoesäure, erhalten durch Carboxylierung eines Alkalimetall-1-naphtholats nach Kolbe-Schmitt und nachfolgende Behandlung mit Wasser, oder durch Auflösen einer auf diese Weise hergestellten, jedoch zwischenisolierten 1-Hydroxy-2-naphthoesäure in wässriger Alkalilauge, mittels Mineralsäure auf einen pH-Wert von 5,5 bis 6,5 einstellt, dann eine oberflächenaktive, kationische Verbindung

(1) der allgemeinen Formel (II)

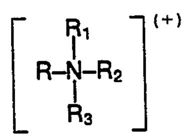

$X^{(-)}$ (II),

in welcher R, $R_1$, $R_2$ und $R_3$ gleiche oder verschiedene, geradkettige oder verzweigte, gesättigte oder 1, 2 oder 3 Kohlenstoffdoppelbindungen enthaltende aliphatische Reste von 1 bis 30 Kohlenstoffatomen, die 1 bis 3 Heteroatome oder Gruppierungen aus der Reihe **–O–**, **–S–**, **–NH–**, **–N–**
**alkyl**
und quaternierte Stickstoffatome, und/oder Carbonsäureamidgruppen, enthalten können, und die ferner an den Kettenenden ionogene Substituenten enthalten können, oder hydroaromatische carbocyclische oder cycloaliphatische Reste von jeweils 4 bis 8 Ringkohlenstoffatomen, wobei diese cyclischen Reste aliphatische Seitenketten von 1 bis 12 Kohlenstoffatomen oder Halogenatome tragen können, oder Aralkylreste, wobei der Alkylrest 1 bis 30 Kohlenstoffatome enthält, und der Arylrest einen Phenyl- oder Naphthylrest darstellt, der am aromatischen Kern durch Hydroxygruppen, niedere Alkylgruppen, niedere Alkoxygruppen und/oder Halogenatome substituiert sein kann, oder Arylreste, die am aromatischen Kern durch Hydroxy-, niedere Alkoxy-, niedere Alkyl-, Carbonamid- oder Sulfonamidgruppen oder Halogenatome substituiert sein können, bedeuten, wobei die Summe der Kohlenstoffatome von R, $R_1$, $R_2$ und $R_3$ mindestens 8 beträgt, und wobei zwei oder drei der am quartären Stickstoffatom befindlichen aliphatischen Reste R, $R_1$, $R_2$ und $R^3$ gemeinsam mit dem Stickstoffatom einen heterocyclischen, ggfs. Doppelbindungen enthaltenden Ring, an welchen ein Benzolkern ankondensiert sein kann, bilden können und in welcher $X^{(-)}$ das Äquivalent einer anorganischen oder organischen Säure darstellt, oder

(2) der allgemeinen Formel (III)

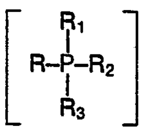 $X^{(-)}$ (III)

in welcher R, $R_1$, $R_2$, $R_3$ und $X^{(-)}$ die bei der Definition der Formel (II) genannten Bedeutungen haben, wobei die Summe der Kohlenstoffatome von R, $R_1$, $R_2$ und $R_3$ mindestens 8 ist und zwei oder drei der aliphatischen Reste R, $R_1$, $R_2$ und $R_3$ mit dem Phosphoratom einen heterocyclischen, ggfs. Doppelbindungen enthaltenden Ring bilden können, jeweils in einer Menge von 0,5 bis 5 Gew.-%, bezogen auf die in der Lösung vorliegende Menge an 1-Hydroxy-2-naphthoesäure, zugibt und die sich dabei in fester oder flüssiger Form ausscheidenden, aus den vorhandenen Harzen in quantitativer Reaktion gebildeten Niederschläge bei Temperaturen von 50 °C bis 100 °C, ggfs. nach Zugabe von Adsorbentien und/oder Filterhilfsmitteln, separiert oder abfiltriert und das in wässriger Lösung vorliegende reine Mono-Alkalimetallsalz der 1-Hydroxy-2-naphthoesäure mit Dialkylsulfat im pH-Bereich von 5,5 bis 6,5 bei Temperaturen zwischen 30 °C und 80 °C verestert.

2. Verfahren nach Anspruch 1, dadurch gekennzeichnet, dass man das nach Abtrennung der aus den vorhandenen Harzen gebildeten Niederschläge in wässriger Lösung anfallende reine Mono-Alkalimetallsalz der 1-Hydroxy-2-naphthoesäure vor der Veresterung mit Dialkylsulfat durch Zugabe von Mineralsäure in Form der 1-Hydroxy-2-naphthoesäure abscheidet und diese nach Neutralwaschen und Trocknen wieder in Natronlauge löst.

## Claims

1. A process for preparing pure alkyl 1-hydroxy-2-naphthoates of the formula (I)

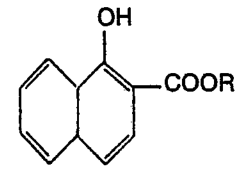 (I)

in which R denotes straight-chain or branched alkyl of 1 to 5 carbon atoms, which comprises adjusting, with mineral acid, to pH 5.5–6.5 an aqueous solution of monoalkali and/or dialkali metal salts of 1-hydroxy-2-naphthoic acid obtained by carboxylating an alkali metal 1-naphtholate by the Kolbe-Schmitt method, followed by treatment with water, or by dissolving, in aqueous alkali metal hydroxide solution, 1-hydroxy-2-naphthoic acid prepared in this way but isolated, then adding a surface-active cationic compound (1) of the general formula (II)

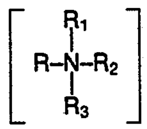 $X^{(-)}$ (II)

in which R, $R_1$, $R_2$ and $R_3$ each denote identical or different, straight-chain or branched aliphatic radicals of 1 to 30 carbon atoms which are saturated or contain 1, 2 or 3 carbon-carbon double bonds and which can contain 1 to 3 hetero atoms or groupings from the series consisting of

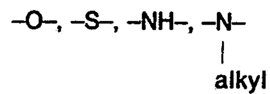

and quaternized nitrogen atoms, and/or carboxamide groups, and which, furthermore, can contain in terminal positions, ionic substituents, or hydroaromatic carbocyclic or cycloaliphatic radicals which each have 4 to 8 ring carbon atoms and can bear aliphatic side chains of 1 to 12 carbon atoms or halogen atoms, or aralkyl radicals where the alkyl radical contains 1 to 30 carbon atoms and the aryl radical is a phenyl or naphthyl radical which can be substituted in the aromatic nucleus by hydroxyl groups, lower alkyl groups, lower alkoxy groups and/or halogen atoms, or aryl radicals which can be substituted on the aromatic nucleus by hydroxyl, lower alkoxy, lower alkyl, carboxamide or sulfonamide groups or halogen atoms, the total number of the carbon atoms of R, $R_1$, $R_2$ and $R_3$ being at least 8 and two or three of the aliphatic radicals R, $R_1$, $R_2$ and $R_3$ on the quaternary nitrogen atom being capable of forming together with the nitrogen atom a heterocyclic ring which may contain double bonds and onto which a benzene nucleus can be fused and in which $X^{(-)}$ represents one equivalent of an inorganic or organic acid, or

(2) of the general formula (III)

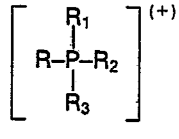 $X^{(-)}$ (III)

in which R, $R_1$, $R_2$, $R_3$ and $X^{(-)}$ have the meanings given in the definition of formula (II), where the total number of carbon atoms of R, $R_1$, $R_2$ and $R_3$ is at least 8 and two or three of the aliphatic radicals R, $R_1$, $R_2$ and $R_3$ can form together with the phosphorus atom a heterocyclic ring which may contain double bonds, in each case in an amount of 0.5 to 5% by weight relative to the amount of 1-hydroxy-2-naphthoic acid present in the solution, separating or filtering off the solid or liquid precipitates quantitatively formed from the resins present at temperatures of 50 °C to 100 °C if desired after addition of adsorbents and/or filtering aids and esterifying with dialkyl sulfate the pure monoalkali metal salt of 1-hydroxy-2-naphthoic acid present in aqueous solution at pH 5.5–6.5 and temperatures between 30 °C and 80 °C.

2. The process as claimed in claim 1, wherein the pure monoalkali metal salt of 1-hydroxy-2-naphthoic acid obtained in aqueous solution after removal of the precipitates formed from the resins present is precipitated, prior to the esterification with dialkyl sulfate, in the form of 1-hydroxy-2-naphthoic acid by addition of mineral acid, and this 1-hydroxy-2-naphthoic acid is washed neutral, dried and redissolved in sodium hydroxide solution.

**Revendications**

1. Procédé de préparation d'esters alkyliques purs de l'acide 1-hydroxy-2-naphtoïque de formule générale I ci-dessous:

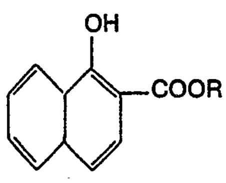

(I),

dans laquelle R désigne un alkyle en $C_1$–$C_5$ à chaîne linéaire ou ramifiée, procédé caractérisé en ce que l'on règle à un pH de 5,5 à 6,5, avec un acide minéral, une solution aqueuse des sels mono- et/ou di-alcalines de l'acide 1-hydroxy-2-naphtoïque obtenue par carboxylation d'un 1-naphtolate de métal alcalin (suivant Kolbe-Schmitt) suivie d'un traitement à l'eau, ou par dissolution dans une lessive alcaline aqueuse d'un acide 1-hydroxy-2-naphtoïque formé de cette manière mais qui a été isolé, puis on ajoute un composé surfactif cationique soit:

(1) de formule générale II:

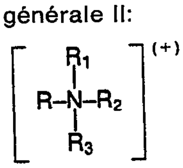

$X^{(-)}$ (II),

dans laquelle R, $R_1$, $R_2$ et $R_3$ représentent des radicaux aliphatiques en $C_1$ à $C_{30}$ identiques ou différents, à chaîne linéaire ou ramifiée, saturés ou avec une, deux trois doubles liaisons carbone-carbone, pouvant avoir de 1 à 3 hétéroatomes ou groupes tels que –O–, –S–, –NH–, 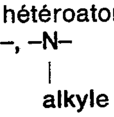 ou des atomes d'azote quaternisés, et/ou des groupes amide carboxylique, ainsi qu'aux extrémités des chaînes des substituants ionogènes, ou bien des radicaux hydroaromatiques carbocycycliques ou cycloaliphatiques ayant de 4 à 8 atomes de carbone dans le cycle, radicaux cycliques qui peuvent porter des chaînes latérales aliphatiques en $C_1$ à $C_{12}$ ou des atomes d'halogènes, par exemple de chlore ou de brome, ou des radicaux aralkyles dont l'alkyle a de 1 à 30 atomes de carbone, de préférence de 1 à 12, et l'aryle est un groupe phényle ou naphtyle dont le cycle peut avoir comme substituants des hydroxyles, des groupes alkyles ou alcoxy inférieures et/ou des atomes d'halogènes, ou encore des radicaux aryles, dont le cycle peut avoir comme substituants des hydroxyles, des alkyles ou des alcoxy inférieurs, des groupes carboxamides ou sulfonamides ou des atomes d'halogènes, par exemple de chlore, la somme des atomes de carbone de R, $R_1$, $R_2$ et $R_3$ étant au moins égale à 8 et 2 ou 3 des radicaux aliphatiques R, $R_1$, $R_2$ et $R_3$ situés sur un atome d'azote quaternaire pouvant former avec l'atome d'azote un hétérocycle éventuellement avec des doubles liaisons, sur lequel peut être condensé un noyau benzénique, et $X^{(-)}$ représente l'équivalent d'un acide minéral ou organique, soit

(2) de formule générale III:

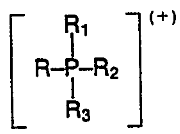

$X^{(-)}$ (III)

dans laquelle les symboles R, $R_1$, $R_2$, $R_3$ et $X^{(-)}$ ont les mêmes significations que dans la formule II ci-dessus, la somme des atomes de carbone de R, $R_1$, $R_2$ et $R_3$ étant au moins égale à 8 et 2 ou 3 des radicaux aliphatiques R, $R_1$, $R_2$ et $R_3$ peuvent former avec l'atome de phosphore un hétérocycle avec éventuellement des doubles liaisons, l'un ou l'autre de ces composés dans une proportion de 0,5 à 5% du poids de l'acide 1-hydroxy-2-naphtoïque se trouvant dans la solution, on élimine ou on sépare par filtration les précipités qui se sont formés à l'état solide ou liquide à partir des résines en une réaction quantitative, à des températures de 50 à 100 °C, éventuellement après avoir ajouté des adsorbants et/ou des adjuvants de filtration, et on estérifie avec un sulfate de dialkyle le sel de métal monoalcalin pur de l'acide 1-hydroxy-2-naphtoïque qui se trouve dans la solution aqueuse à un pH de 5,5 à 6,5, à des températures de 30 à 80 °C.

2. Procédé selon la revendication 1 caractérisé en ce que, après avoir séparé les précipités formés à partir des résines présentes, avant d'effectuer l'estérification avec le sulfate de dialkyle, on fait précipiter à l'état d'acide 1-hydroxy-2-naphtoïque le sel monoalcalin pur de cet acide obtenu en solution aqueuse en ajoutant un acide minéral, on lave cet acide 1-hydroxy-2-naphtoïque jusqu'à neutralité, on le sèche et on le redissout dans une lessive de soude.